# EUROPEAN PATENT SPECIFICATION

(11) **EP 3 840 879 B1**
(45) Date of publication and mention of the grant of the patent: **03.09.2025**
(21) Application number: 19752798.9
(22) Date of filing: 30.07.2019
(51) Int. Cl.: B01L 3/00, G01N 21/07

(54) **DEVICES WITH OPTICALLY READABLE LIQUID RESERVOIRS**
VORRICHTUNGEN MIT OPTISCH LESBAREN FLÜSSIGKEITSRESERVOIREN
DISPOSITIFS COMPRENANT DES RÉSERVOIRS DE LIQUIDE LISIBLES OPTIQUEMENT

(30) Priority: 23.08.2018 US 201862722029 P; 18.07.2019 US 201916516001
(43) Date of publication of application: 30.06.2021
(73) Proprietor: Truvian Sciences, Inc., San Diego, CA 92121 (US)
(72) Inventor: KLEINEMOLEN, Ian, San Diego, CA 92121 (US); MARRINUCCI, Dena, San Diego, CA 92121 (US); ABI-SAMRA, Kameel, Michael, San Diego, CA 92121 (US); HAWKINS, Jeffrey A., San Diego, CA 92121 (US)
(74) Representative: Carrie, Rosemary Louise Hunter
(86) International application number: PCT/US2019/044038
(87) International publication number: WO 2020/040946

(56) References cited:
- EP-A1- 2 116 305
- EP-A2- 0 163 063
- WO-A1-2017/027384
- DE-A1- 19 858 443

## Description

### RELATED APPLICATIONS

The current application claims priority to U.S. Patent Application No. 16/516,001 filed July 18, 2019 which, in turn, claims priority to U.S. Patent Application No. 62/722,029 filed on August 23, 2018.

### FIELD

This application relates to devices with liquid reservoirs.

### BACKGROUND

It can be useful to read out information optically from liquid samples, for example by shining laser light into a liquid sample and sensing light from the liquid sample, wherein information about the sample can be determined from the sensed light.
EP0163063 describes a multicuvette rotor for use in a centrifugal analyzer that includes a rotor drive for rotating the rotor at rates greater than one thousand rpm for mixing reactants and performing analyses while the rotor is being driven in rotation defines a circumferential array of elongated radially extending cuvettes. Each elongated cuvette defines a first chamber for receiving a first reactant and a loading port through which the first reactant is introduced into the first chamber region, a second chamber region for receiving a second reactant and a loading port through which the second reactant is introduced into the second chamber region, and divider structure between the first and second chamber regions that provides a transfer passage between the first and second chamber regions through which the first reactant may be flowed into the second chamber region for forming a reaction product with the second reactant. An analysis region is defined adjacent the radially outer wall of each cuvette where the resulting reaction product is subjected to analysis, the analysis regions being disposed in a circumferential array adjacent the periphery of the rotor, and mechanical interlock structure that is radially aligned with the analysis regions is adapted to receive a thermal sensor carried by the rotor drive for monitoring analysis region temperature during data acquisition.

### SUMMARY

Devices with optically readable liquid reservoirs, and methods of making and using the same, are provided herein. The invention to which this European patent relates is defined by the appended claims.

The device includes a lower reservoir surface, an upper reservoir surface, and a reservoir sidewall extending between the upper and lower reservoir surfaces which together define a reservoir. The reservoir is configured to be completely filled by a liquid such that the liquid forms a column contacting the upper reservoir surface, the lower reservoir surface, and the reservoir sidewall, with a meniscus of the liquid being outside of the reservoir. At least one of the upper reservoir surface and the lower reservoir surface is configured to transmit light.

A channel is coupled to the reservoir sidewall. With such an arrangement, the meniscus can be located within the channel. A well fluidically can be coupled to the reservoir via the channel such that the meniscus is located within the well.

The assay chamber is fluidically coupled to the reservoir via the channel. The assay chamber includes an inlet. The assay chamber can have a reagent disposed therein.
The reagent can be configured to react with liquid received in the assay chamber via the inlet. The channel can be configured to transmit the liquid from the assay chamber to the reservoir responsive to application of a force (e.g., centrifugal force, a gas source, etc.) to the assay chamber.

A rotatable disc can be provided in which the reservoir is disposed. Rotating such disc can generate centrifugal force.

The assay chamber includes a lower assay chamber surface, an upper assay chamber surface, and an assay chamber sidewall extending between the upper and lower assay chamber surfaces. The assay chamber sidewall can include a first portion extending substantially perpendicularly to the upper and lower assay chamber surfaces. The assay chamber sidewall includes a portion extending at an obtuse angle, from the lower assay chamber surface.

The liquid can be conveyed upward along the second portion and into the channel responsive to application of force.

In some variations, the assay chamber sidewall and the reservoir sidewall can be integrally formed with one another. In other variations, the upper assay chamber surface and the upper reservoir surface can be integrally formed with one another and attached to the integrally formed assay chamber sidewall and the reservoir sidewall. In still other variations, the lower assay chamber surface and the lower reservoir surface can be integrally formed with one another and attached to the integrally formed assay chamber sidewall and the reservoir sidewall. In further variations, all of the assay chamber sidewall, the channel, and the reservoir sidewall can be integrally formed with one another.

Further, the assay chamber sidewall and the reservoir sidewall can be discrete elements. The upper assay chamber surface and the upper reservoir surface can be discrete elements. The lower assay chamber surface and the lower reservoir surface can be discrete elements. The assay chamber sidewall, the channel, and the reservoir sidewall can be discrete elements.

The channel and the sidewall can be integrally formed with one another. The channel and the sidewall can be discrete elements.

The lower reservoir surface, the upper reservoir surface, and the sidewall can be discrete elements attached to one another.

The reservoir sidewall can define a circular, rectangular, square, or irregular cross section of the reservoir.

The device can include a source of light such as, for example, a laser, light emitting diode, or lamp. The source of the light can be positioned over the upper reservoir surface and configured to transmit the light through (e.g., laterally through, etc.) the upper reservoir surface. The source of the light further can be configured to transmit the light through the column and then through the lower reservoir surface. The source of the light can be positioned under the lower reservoir surface and be configured to transmit the light through the lower reservoir surface. The source of the light further can be configured to transmit the light through the column and then through the upper reservoir surface.

The device can include a sensor configured to receive (and characterize) the light transmitted through the at least one of the upper reservoir surface and the lower reservoir surface. The sensor can be positioned in a variety of locations. For example, the sensor can be positioned over the upper reservoir surface and be configured to receive the light through the upper reservoir surface. The sensor can be positioned under the lower reservoir surface and be configured to receive the light through the lower reservoir surface.

The light can be generated by, for example, fluorescence or chemiluminescence.

Reagents that can be used with the device include an antibody, enzyme, or particle.

The reservoir can have varying volumes. For example, the reservoir can have a volume of about 1-200 µL, or about 10-100 µL, or about 15-50 µL, or about 10-30 µL, or about 5-20 µL.

The device can house or otherwise characterize a wide variety of liquids. For example, the liquid can be a bodily fluid such as whole blood, blood plasma, blood cells, urine, and/or spit. The liquid can be a food sample, a water sample, a purified nucleic acid, a pharmaceutical compound, a buffer, and/or a reagent.

In another aspect, a reservoir can be filled (e.g., completely filled, substantially filled, etc.) with a liquid. The reservoir can include a lower reservoir surface, an upper reservoir surface, and a reservoir sidewall extending between the upper and lower reservoir surfaces. The liquid forms a column contacting the upper reservoir surface, the lower reservoir surface, and the reservoir sidewall. A meniscus of the liquid is located outside of the reservoir. Light is transmitted through at least one the upper reservoir surface and the lower reservoir surface.

### BRIEF DESCRIPTION OF DRAWINGS

FIGS. 1A-1B respectively schematically illustrate cross-sectional and plan views of an exemplary device with an optically readable liquid reservoir, not forming part of the invention.
FIGS. 2A-2C schematically illustrate cross-sectional views of exemplary devices with an optically readable liquid reservoir, a light sensor, and an optional light source, not forming part of the invention.
FIGS. 3A-3B schematically illustrate cross-sectional views of exemplary devices with wells attached to optically readable reservoirs, not forming part of the invention.
FIGS. 4A-4C schematically illustrate views of alternative devices with an optically readable liquid reservoir, not forming part of the invention.
FIGS. 5A-5C schematically illustrate perspective and plan views of components of an alternative device with an optically readable liquid reservoir, according to various configurations provided herein.
FIG. 6 schematically illustrates a plan view of a device including multiple of the devices of FIGS. 5A-5C, according to various configurations provided herein.
FIG. 7 illustrates an exemplary flow of operations in a method of using the devices of FIGS. 5A-C, 6 according to various configurations provided herein.

### DETAILED DESCRIPTION

Devices with optically readable liquid reservoirs, and methods of making and using the same, are provided herein. The present devices can facilitate obtaining information from liquid samples by providing a reservoir that can be completely filled with liquid such that a meniscus of the liquid is outside of the reservoir. Location of the meniscus outside of the reservoir can facilitate reading out information optically from the sample within that reservoir. For example, the liquid can form a column within the reservoir that is bounded by top and bottom surfaces and a sidewall of the reservoir. At least one of top and bottom surfaces is at least partially transparent, thus permitting sensing of light from or through the liquid and through the partially transparent top and/or bottom surface(s) without that light being transmitted through the meniscus. As a comparison, transmission of such light through a meniscus can alter the path focus, and other qualities of the light, which can hinder read out of information.

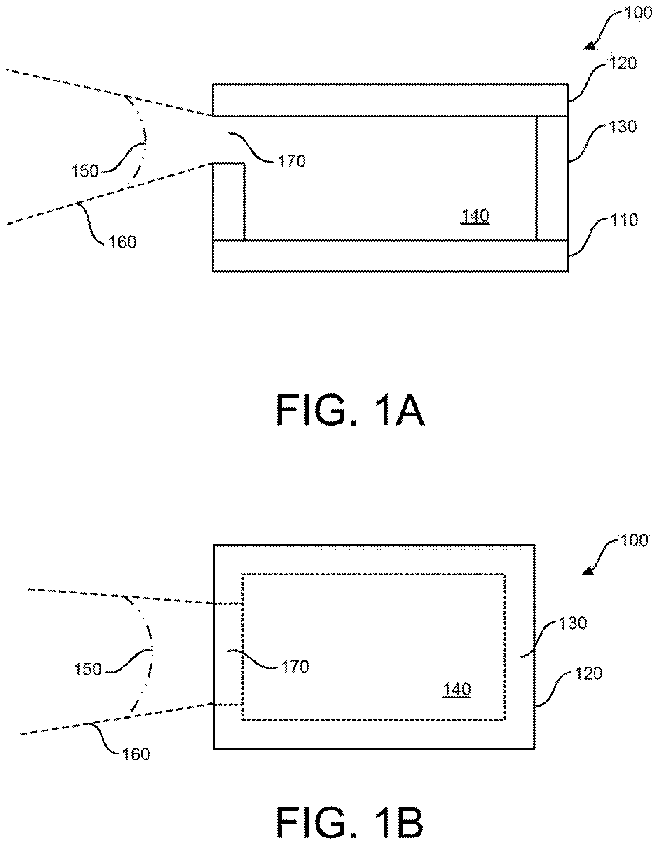

FIGS. 1A-1B respectively schematically illustrate cross-sectional and plan views of an exemplary device not forming part of the invention with an optically readable liquid reservoir. In FIGS. 1A-1B, device 100 includes lower reservoir surface 110, upper reservoir surface 120, and one or more reservoir sidewalls 130 extending between the upper and lower reservoir surfaces 110. Lower reservoir surface 110, upper reservoir surface 120, and reservoir sidewall(s) 130 define reservoir 140. As shown in FIGS. 1A-1B, reservoir 140 is configured to be completely filled by a liquid such that the liquid forms a column contacting upper reservoir surface 120, lower reservoir surface 110, and reservoir sidewall 130, with a meniscus 150 of the liquid being outside of the reservoir. By "completely filled" it is meant that the liquid contacts substantially the entirety of reservoir 140, e.g., substantially completely and directly contacts upper surface 120, substantially completely and directly contacts lower surface 110, and substantially completely and directly contacts sidewall(s) 130. Such substantially complete and direct contact between the liquid and the surface can include a relatively small area, for example 10% or less of that surface's area, in which a bubble or particle is interposed between the liquid and that surface. Additionally, it should be appreciated that the liquid and meniscus 150 optionally need not be considered to form part of the present device 100.

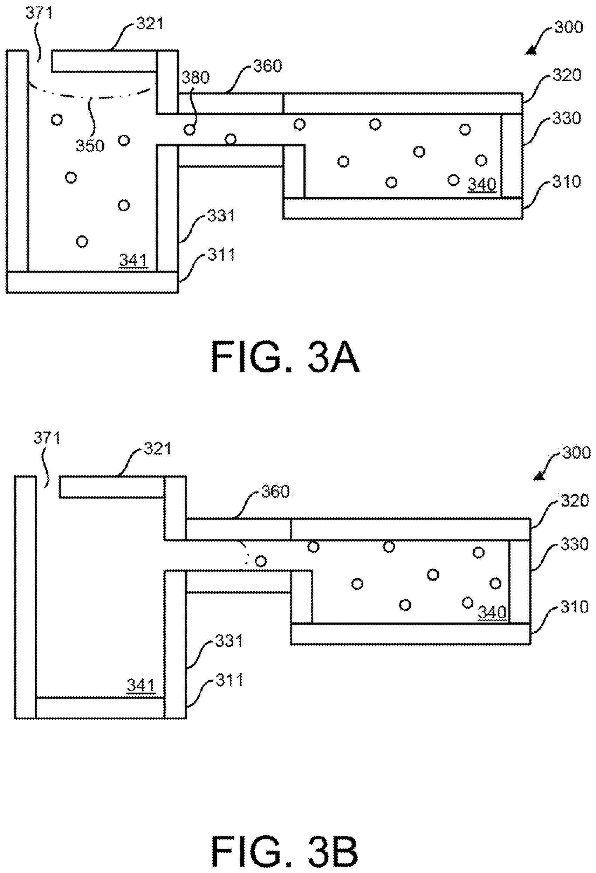

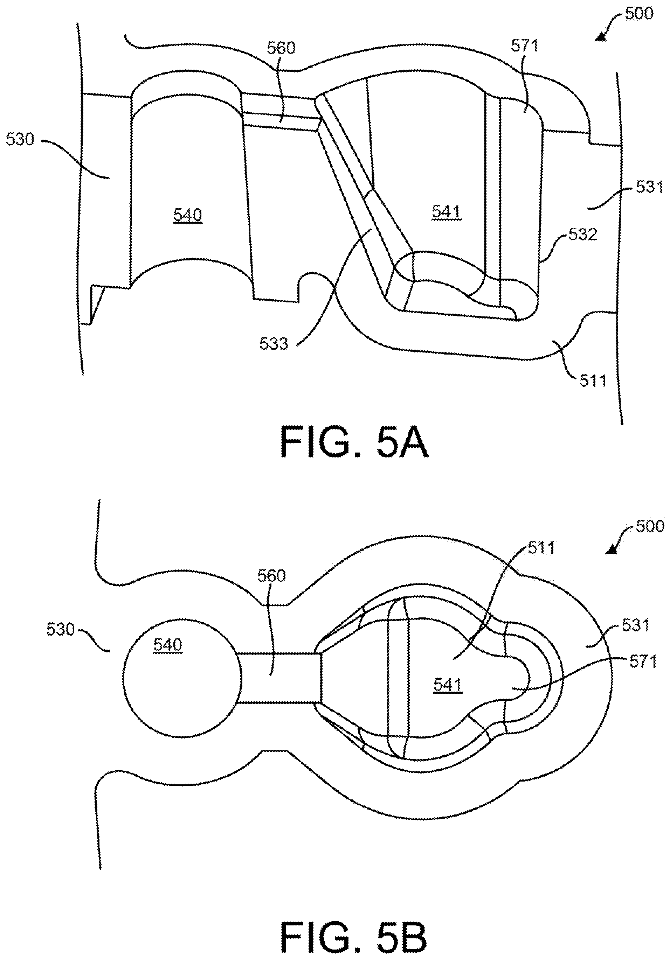

In FIGS. 1A-1B, an optional structure 160 is fluidically coupled to reservoir 140, e.g., via an opening 170 through sidewall(s) 130, and configured to receive meniscus 150. In FIG. 1B, the boundary of reservoir 140 and opening 170 are indicated in dotted lines. It should be appreciated that structure 160 can be, but need not necessarily be considered to form part of the present device 100. Structure 160 can have any suitable configuration for receiving meniscus 150, for example but not limited to examples such as described in greater herein with reference to FIGS. 3A-3B and 5A-5C.

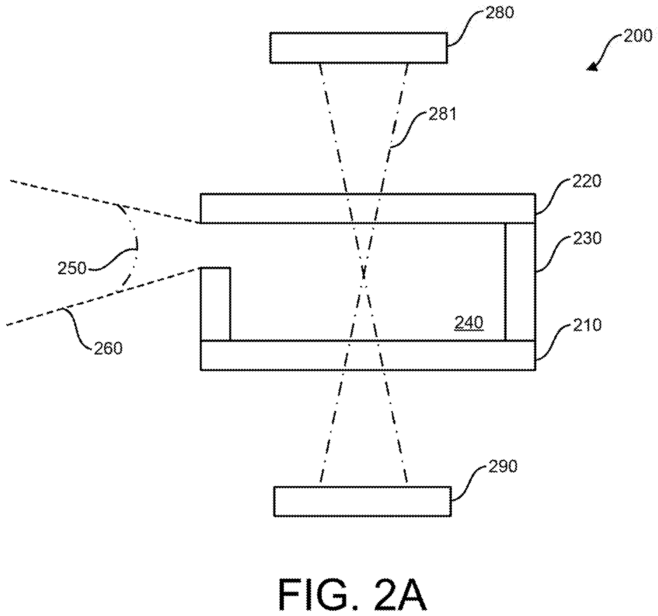

In configurations such as illustrated in FIGS. 1A-1B, at least one of the upper reservoir surface 120 and lower reservoir surface 110 is configured to transmit light. As such, light from or through the top and/or bottom of the column of liquid within reservoir 140 can be sensed from outside of the reservoir so as to obtain information about that liquid. For example, FIGS. 2A-2C schematically illustrate cross-sectional views of exemplary devices with an optically readable liquid reservoir, a light sensor, and an optional light source, not forming part of the invention.

In the exemplary configuration illustrated in FIG. 2A, device 200 includes lower reservoir surface 210, upper reservoir surface 220, and reservoir sidewall(s) 230 defining a reservoir 240 configured similarly as described with reference to FIGS. 1A-1B. Device 200 optionally also can include source 280 of light, which can be configured so as to transmit light into reservoir 240 via the upper reservoir surface 220 or lower reservoir surface 210. Examples of source 280 of light include, but are limited, to a laser, light emitting diode, or lamp (such as a halogen, mercury, or xenon lamp). The light generated by source 280 can be narrowband or broadband, and can be coherent or incoherent. Additionally, the light can have any suitable wavelength(s), for example in the infrared, visible, or ultraviolet regions of the spectrum. In configurations such as illustrated in FIG. 2A, source 280 of the light optionally is positioned over upper reservoir surface 220 and configured to transmit the light through the upper reservoir surface 220. Additionally, as illustrated in FIG. 2A, source 280 of the light further optionally can be configured to transmit the light through the column of liquid within reservoir 240 and then through the lower reservoir surface 210. In such configurations, both upper reservoir surface 220 and lower reservoir surface 210 can be at least partially optically transparent. Additionally, it should be appreciated that source 280 can be located at any suitable position relative to reservoir 240. For example, in an alternative configuration, source 280 of the light optionally can be positioned under the lower reservoir surface 210 and configured to transmit the light through the lower reservoir surface 210. As a further option of such a configuration, source 280 of the light further can be configured to transmit the light through the column of liquid within reservoir 270 and then through the upper reservoir surface 220. In such configurations, both upper reservoir surface 220 and lower reservoir surface 210 can be at least partially optically transparent.

Referring still to the exemplary configuration illustrated in FIG. 2A, device 200 optionally further can include sensor 290 configured to receive the light transmitted through the upper reservoir surface 220 and/or lower reservoir surface 210. Sensor 290 can have any suitable configuration, such as a photodetector, photodiode, photomultiplier, charge coupled device, and the like, and can be configured to generate an electrical signal based on light received from or through the liquid within reservoir 240. Information about the liquid can be obtained based on such an electrical signal. For example, in FIG. 2A, sensor 290 is positioned under the lower reservoir surface 210 and is configured to receive the light through the lower reservoir surface 210, which light can be generated by source 280. In an alternative configuration such as noted above in which source 280 of the light is positioned under lower reservoir surface 210, sensor 290 optionally can be positioned over the upper reservoir surface 220 and configured to receive the light through the upper reservoir surface 220. In any such configuration, note that meniscus 250 is located outside of reservoir 240, e.g., within structure 260. As such, meniscus 250 is outside of the optical path 281 between source 280 and sensor 290 and therefore does not interfere with optically obtaining information about the liquid.

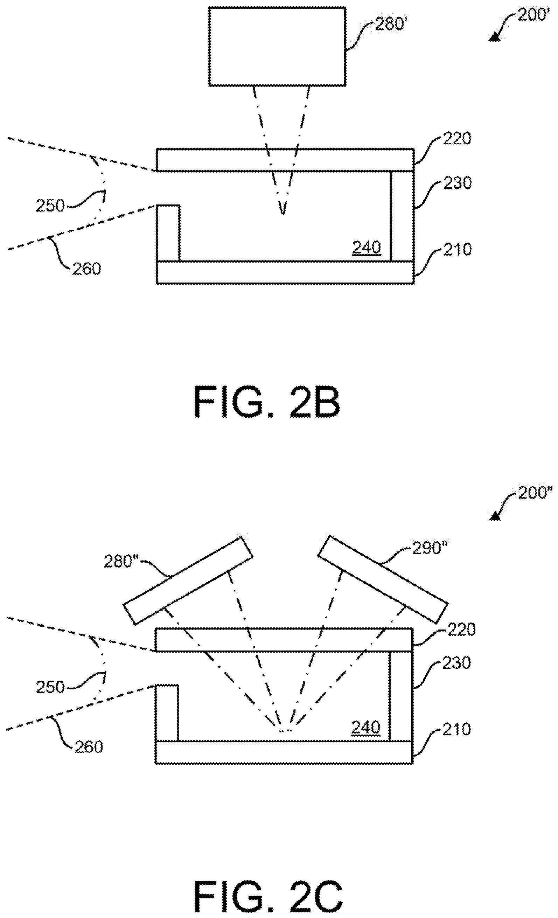

Note that information about the liquid can be obtained in a variety of suitable configurations, not all of which require "transmission-mode" arrangements of the light source and/or sensor such as described above with reference to FIG. 2A, which can be considered to be arrangements. For example, FIG. 2B illustrates an exemplary device 200', not forming part of the invention, including element 280' located above upper reservoir surface 220 and configured to transmit light to and/or receive light from liquid within reservoir 240 only through upper reservoir surface 220. Alternatively, element 280' can be located below lower reservoir surface 210 and configured to transmit light to and/or receive light from liquid within reservoir 240 only through lower reservoir surface 210. For example, element 280' optionally can include a light source (which can be configured similarly as light source 280 described with reference to FIG. 2A) and/or optionally can include a sensor (which can be configured similarly as sensor 290 described with reference to FIG. 2A). In configurations where element 280' includes both a light source and a sensor, element 280' optionally can include at least one optic that both transmits light from the light source and receives light from the liquid within reservoir 240. In one exemplary configuration, element 280' is or includes a confocal microscope.

As another example, FIG. 2C illustrates an exemplary device 200", not forming part of the invention, including source 280" of light and sensor 290", both of which are located above upper reservoir surface 220 and respectively configured to transmit light to and receive light from liquid within reservoir 240 only through upper reservoir surface 220. Alternatively, source 280" of light and sensor 290" both can be located below lower reservoir surface 210 and respectively configured to transmit light to and receive light from liquid within reservoir 240 only through lower reservoir surface 210. Source 280" can be configured similarly as light source 280 described with reference to FIG. 2A, and sensor 290" can be configured similarly as sensor 290 described with reference to FIG. 2A. The configuration illustrated in FIG. 2C can be considered to be a "reflection-mode" configuration.

In some configurations such as exemplified by FIGS. 2A-2C, light can be generated by a suitable light source, e.g., 280, 280', or 280" and can be received by a suitable sensor, e.g., 290, 280', or 290" which generates an electrical signal based upon which information about the liquid within reservoir 240 can be obtained. For example, the light can be partially or fully absorbed by the liquid within reservoir 240 generating an interpretable signal within the electrical signal generated by the sensor, and information about the liquid can be obtained by analyzing the electrical signal. As another example, the light can cause the liquid within reservoir to fluoresce, such fluorescence generating an interpretable signal within the electrical signal generated by the sensor, and information about the liquid can be obtained by analyzing the electrical signal. However, a light source need not necessarily be required in order to obtain information about the liquid within reservoir 240 via light that the sensor receives. For example, the light can be generated by chemiluminescence of the liquid, such chemiluminescence generating an interpretable signal within the electrical signal generated by the sensor, and information about the liquid can be obtained by analyzing the electrical signal. Other suitable configurations for obtaining and analyzing light from liquid within reservoir 240 can be implemented.

As noted above with reference to FIGS. 1A-1B, the present devices can include or be coupled to another structure 160 within which the liquid's meniscus can be located. For example, FIGS. 3A-3B schematically illustrate cross-sectional views of exemplary devices with wells attached to optically readable reservoirs, according to various configurations provided herein. In the exemplary configuration illustrated in FIGS. 3A-3B, device 300 includes lower reservoir surface 310, upper reservoir surface 320, and reservoir sidewall(s) 330 defining a reservoir 340 configured similarly as described with reference to FIGS. 1A-1B. Device 300 optionally also can include a source of light and/or sensor configured similarly as described with reference to FIGS. 2A-2C.

As illustrated in FIGS. 3A-3B, device 300, not forming part of the invention, optionally can include channel 360 coupled to reservoir sidewall 330. In some configurations, device 300 optionally further includes a well 341 that is fluidically coupled to the reservoir 340 via the channel 360. The meniscus 350 can have any suitable location within device 300. For example, in some configurations such as illustrated in FIG. 3A, meniscus 350 is located within the well 341, while in other configurations such as illustrated in FIG. 3B, meniscus 350 is located within the channel 360.

Optionally, well 341 can be or include an assay chamber. By "assay chamber" it is meant a reservoir in which a liquid can be assayed, e.g., mixed with one or more reagents with which the liquid chemically and/or biologically reacts to generate a change in the liquid that can be detected optically (e.g., using a sensor such as described with reference to FIGS. 2A-2C). For example, in configurations such as illustrated in FIGS. 3A-3B, well 341 can define an assay chamber 341 that is fluidically coupled to reservoir 340 via channel 360. The assay chamber optionally can include an inlet 371, and as a further option can include a reagent 380 within the assay chamber 341. Reagent 380 can be configured to react with liquid which is received in the assay chamber 341 via the inlet 371. The reagent 380 can be dry or wet prior to addition of the liquid via inlet 371. As illustrated in FIGS. 3A-3B, reagent 380 can be dispersed throughout or dissolved in the liquid. Exemplary reagents include, but are not limited to, an antibody, enzyme, or particle. However, note that use of reagent is optional, in which case element 341 may be considered simply to be a well.

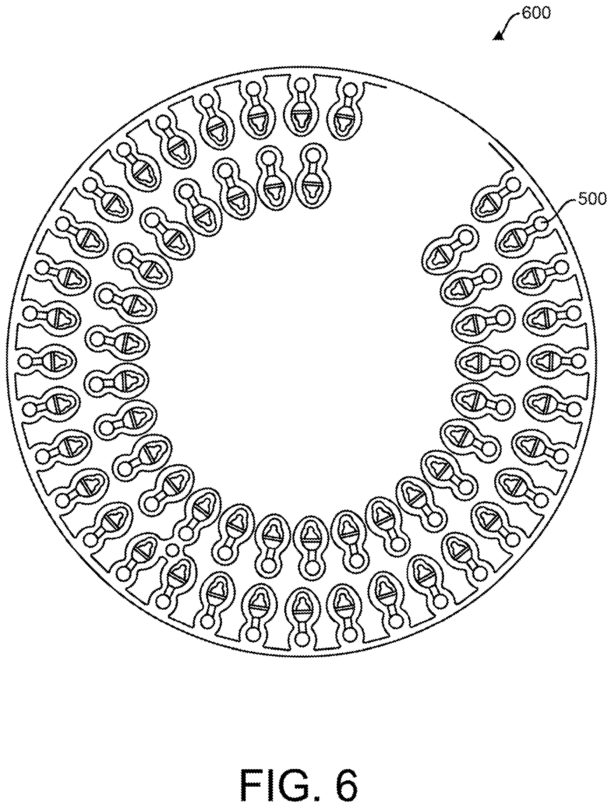

Note that liquid added into assay chamber 341 (which also can be considered a well), e.g., via inlet 371, may not necessarily flow under its own power into reservoir 340 via channel 360. In some configurations, channel 360 is configured to convey the liquid from the assay chamber to the reservoir responsive to application of a force to assay chamber 341. For example, device 300 can include a source of gas (not specifically illustrated) configured to apply the force via the gas. Such gas can be introduced to assay chamber 341 via inlet 371 and can force liquid through channel 360 and into reservoir 340 so as to completely fill the reservoir in a manner such as described with reference to FIGS. 1A-1B. In other exemplary configurations, the force can include a centrifugal force. For example, device 300 can include a rotatable disc in which reservoir 340 is disposed, wherein rotating the disc generates the centrifugal force. An exemplary rotatable disc is described herein with reference to FIG. 6.

In the nonlimiting configuration illustrated in FIGS. 3A-3B, assay chamber 341 (which also can be considered a well) can include lower assay chamber surface 311, upper assay chamber surface 321, and assay chamber sidewall(s) 331 extending between the upper and lower assay chamber surfaces 321, 311. Any suitable combination of lower assay chamber surface 311, upper assay chamber surface 321, assay chamber sidewall(s) 331, channel 360, lower reservoir surface 310, upper reservoir surface 320, and reservoir sidewall(s) 330 can be formed as discrete elements that are attached to one another, or can be integrally formed with one another, and can have any suitable shape and dimensions. In a nonlimiting example, reservoir 340 and/or well 341 each can have a volume of about 1-200 µL, or about 10-100 µL, or about 15-50 µL, or about 10-30 µL, or about 5-20 µL. As used herein, "about" means within 10% of the stated value.

Note that in configurations in which meniscus 350 is located within well 341 such as shown in FIG. 3A, the meniscus can be oriented substantially parallel to the upper and/or lower surfaces 321, 311, and can have a surface area similar to that of the lower surface 311 and/or upper surface 321 as a result of gravitational effects. In comparison, in configurations in which meniscus 350 is located within channel 360 such as shown in FIG. 3B, the meniscus can be oriented substantially perpendicularly to the length of the channel, and can have a surface area similar to that of the height and width of the channel as result of surface tension and capillary action. In such a configuration, the liquid can have a smaller meniscus and can experience a lower rate of evaporation in the configuration of FIG. 3B relative to that in the configuration of FIG. 3A.

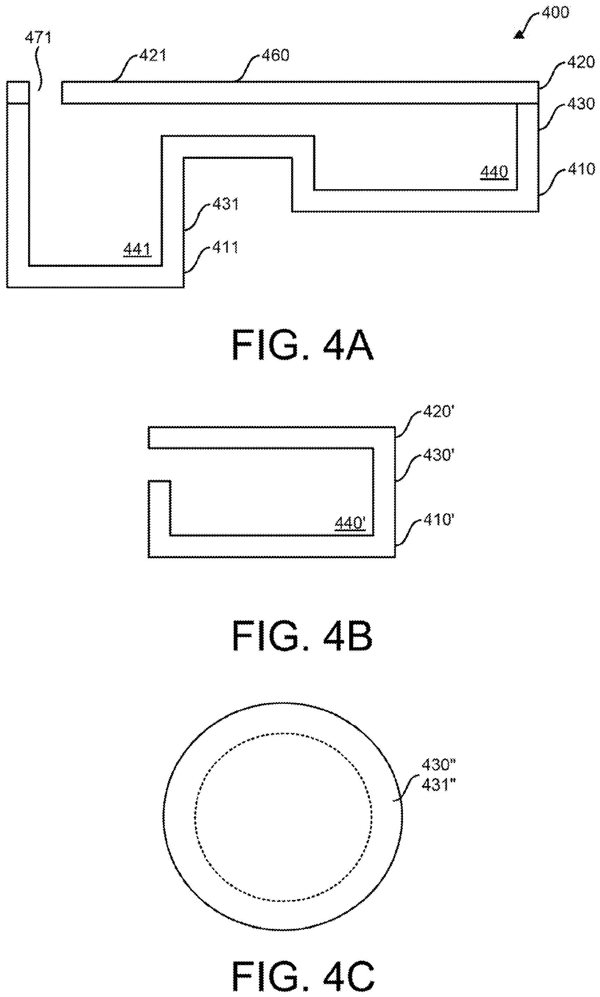

FIGS. 4A-4C schematically illustrate views of alternative devices with an optically readable liquid reservoir, not according to the invention. In the nonlimiting configuration of device 400 illustrated in FIG. 4A, lower assay chamber surface 411, assay chamber sidewall(s) 431, lower surface of channel 460, lower reservoir surface 410, and reservoir sidewall(s) 330 are integrally formed with one another, while upper assay chamber surface 421, upper surface of channel 460, and upper reservoir surface 420 are integrally formed with one another. Referring again to FIGS. 3A-3B, assay chamber sidewall(s) 331 and reservoir sidewall(s) 330 optionally can be integrally formed with one another in a manner similar to that of assay chamber sidewall(s) 431 and reservoir sidewall(s) 430, while other suitable components of device 300 can be integrally formed with one another or discrete from one another For example, the upper assay chamber surface 321 and the upper reservoir surface 320 optionally can be integrally formed with one another in a manner such as illustrated in FIG. 4A and can be attached to such an integrally formed assay chamber sidewall(s) 331 and reservoir sidewall(s) 330. As another example, lower assay chamber surface 311 and lower reservoir surface 310 can be integrally formed with one another in a manner such as illustrated in FIG. 4A and attached to such an integrally formed assay chamber sidewall(s) 331 and reservoir sidewall(s) 330. In still other examples, assay chamber sidewall 331, one or more surfaces of channel 360, and reservoir sidewall 330 can be integrally formed with one another in a manner such as illustrated in FIG. 4A. In yet other configurations, assay chamber sidewall(s) 331 and reservoir sidewall(s) 330 optionally can be discrete elements in a manner such as illustrated in FIGS. 3A-3B. As a further option, upper assay chamber surface 321 and upper reservoir surface 320 can be discrete elements and/or lower assay chamber surface 311 and lower reservoir surface 310 can be discrete elements in a manner such as illustrated in FIGS. 3A-3B. Additionally or alternatively, optionally assay chamber sidewall(s) 331, one or more surfaces of channel 360, and reservoir sidewall(s) 330 can be discrete elements in a manner such as illustrated in FIGS. 3A-3B.

Referring still to FIGS. 3A-3B, note that channel 360 and assay chamber (well) 341 each are optional. If present, such features can be integrally formed with, or discrete from, one or more features of reservoir 340 which features also can be integrally formed with, or discrete from, one another. For example, one or more surfaces (and optionally all surfaces) of optional channel 360 and sidewall 330 can be integrally formed with one another, or can be discrete elements. In the nonlimiting configuration illustrated in FIG. 4B, lower reservoir surface 410', upper reservoir surface 420', and reservoir sidewall(s) 430' are all formed integrally with one another. In another configuration, lower reservoir surface 410', upper reservoir surface 420', and sidewall(s) 430' are discrete elements attached to one another in a manner such as illustrated in FIGS. 3A-3B.

Additionally, reservoir sidewall(s) and assay chamber (well) sidewall(s) such as provided herein can have any suitable cross section. For example, the sidewall(s) of the reservoir and/or assay chamber can define a circular, rectangular, square, or irregular cross section of the reservoir and/or assay chamber. A non-limiting example such sidewall(s) defining a rectangular cross-section is illustrated in FIG. 1B, and such a cross-section similarly can be defined by the sidewall(s) of the assay chamber (well). FIG. 4C illustrates a circular cross section that can be defined by sidewall(s) of the reservoir 430" and/or assay chamber 431".

Still other variations of the present devices readily can be envisioned. For example, FIGS. 5A-5C schematically illustrate perspective and plan views of components of an alternative device 500 with an optically readable liquid reservoir, according to various configurations provided herein. In the exemplary configuration illustrated in FIGS. 5A-5C, device 500 includes a lower reservoir surface (not specifically illustrated), upper reservoir surface (not specifically illustrated), and reservoir sidewall(s) 530 defining a reservoir 540 configured similarly as described with reference to FIGS. 1A-1B. Device 500 optionally also can include a source of light and/or sensor configured similarly as described with reference to FIGS. 2A-2C.

As illustrated in FIGS. 5A-5C, device 500 optionally can include channel 560 coupled to reservoir sidewall 530. In some configurations, device 500 optionally further includes a well 541 that is fluidically coupled to the reservoir 540 via the channel 560. The meniscus (not specifically illustrated) can have any suitable location within device 500. For example, in some configurations similar to those illustrated in FIG. 3A, the meniscus is located within the well 541, while in other configurations similar to those illustrated in FIG. 3B, meniscus 350 is located within the channel. Optionally, well 541 can be or include an assay chamber configured in a manner similar to that described with reference to FIGS. 3A-3B. The assay chamber optionally can include an inlet 571, and as a further option can include a reagent (not specifically illustrated) within the assay chamber 541 which is configured to react with liquid which is received in the assay chamber 541 via the inlet 571.

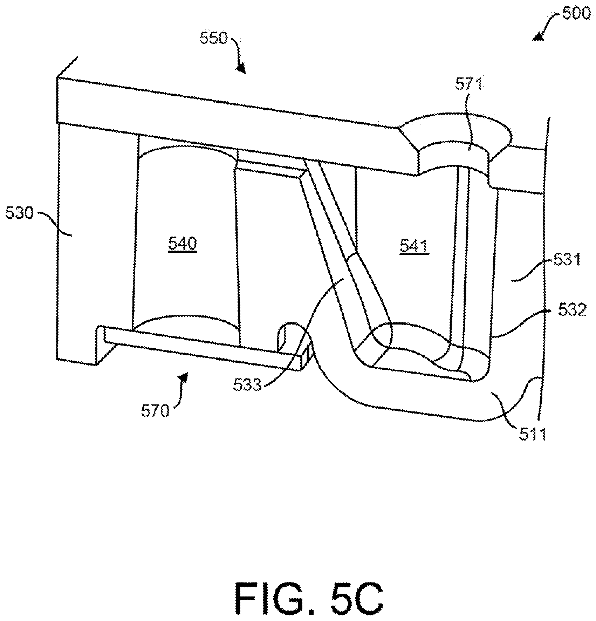

In the nonlimiting configuration illustrated in FIGS. 5A-5C, assay chamber 541 (which also can be considered a well) can include lower assay chamber surface 511, upper assay chamber surface (not specifically illustrated), and assay chamber sidewall(s) 531 extending between the upper and lower assay chamber surfaces. Any suitable combination of lower assay chamber surface 511, upper assay chamber surface (not specifically illustrated), assay chamber sidewall(s) 531, channel 360, lower reservoir surface (not specifically illustrated), upper reservoir surface (not specifically illustrated), and reservoir sidewall(s) 530 can be formed as discrete elements that are attached to one another or can be integrally formed with one another in a manner such as described with reference to FIGS. 3A-3B and 4A-4B, and can have any suitable shape and dimensions in a manner such as described with reference to FIGS. 1A-1B and 4C. In some variations, the device 500 can include a cover 550 and/or a bottom surface 570 as shown in FIG. 5C.

In the exemplary configuration illustrated in FIGS. 5A-5C, assay chamber sidewall 531 optionally includes a first portion 532 extending substantially perpendicularly to the upper and lower assay chamber surfaces. Assay chamber sidewall 531 includes a second portion 533 extending at an obtuse angle, from the lower assay chamber surface 511. Responsive to application of a force such as described herein with reference to FIGS. 3A-3B, liquid that is deposited within assay chamber 541 can be conveyed upward along the second portion and into the channel. For example, FIG. 6 schematically illustrates a plan view of a device 600 including multiple of the devices 500 of FIGS. 5A-5C, according to various configurations provided herein. More specifically, devices 500 can be disposed within a rotatable disc configured so as to be centrifugally spun at a sufficient rate to transfer liquid disposed within assay chamber 541 into reservoir 540 for optical analysis.

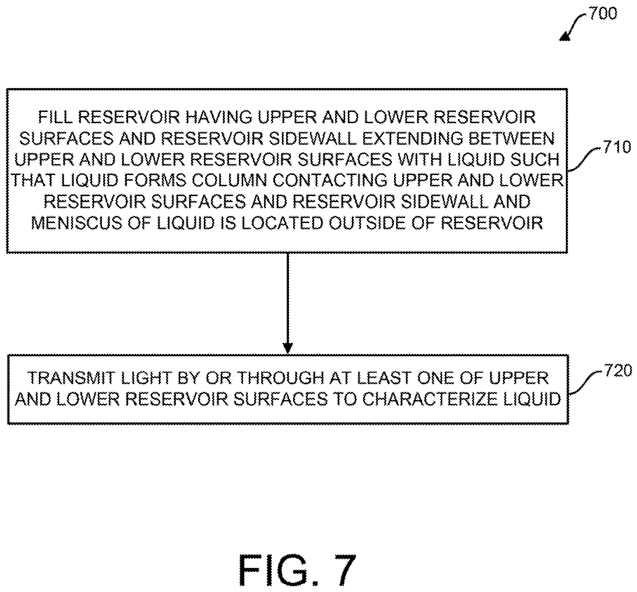

FIG. 7 illustrates an exemplary flow of operations in a method of using the devices of FIGS. 1A-6, according to various configurations provided herein. Method 700 illustrated in FIG. 7 can include completely filling a reservoir with a liquid (710). The reservoir can include a lower reservoir surface; an upper reservoir surface; and a reservoir sidewall extending between the upper and lower reservoir surfaces, e.g., such as described with reference to FIGS. 5A-5C. During operation 710, the liquid forms a column contacting the upper reservoir surface, the lower reservoir surface, and the reservoir sidewall, and a meniscus of the liquid can be located outside of the reservoir, e.g., such as described with reference to FIGS. 1A-1B, 3A-3B, 4A-4C, and 5A-5C. Method 700 illustrated in FIG. 7 also includes transmitting light through at least one the upper reservoir surface and the lower reservoir surface (720), for example such as described with reference to FIGS. 2A-2B.

The device used in method 700 can has a configuration and combination of features such as described with reference to FIGS. 5A-C, 6 A channel is coupled to the reservoir sidewall. The meniscus optionally can be located within the channel. In various optional configurations, the channel and the sidewall can be integrally formed with one another, or can be discrete elements. Additionally, or alternatively, optionally the lower reservoir surface, the upper reservoir surface, and the sidewall are discrete elements attached to one another. Additionally, or alternatively, the reservoir sidewall defines a circular, rectangular, square, or irregular cross section. In various optional configurations, the reservoir has a volume of about 1-200 µL, or about 10-100 µL, or about 15-50 µL, or about 10-30 µL, or about 5-20 µL.

In some optional configurations, a well optionally can be fluidically coupled to the reservoir via the channel, wherein the meniscus optionally can be located within the well. An assay chamber is fluidically coupled to the reservoir via the channel. The assay chamber includes an inlet. A reagent optionally can be within the assay chamber. Optionally, the reagent includes an antibody, enzyme, or particle.

In some configurations, method 700 optionally further includes receiving the liquid in the assay chamber via the inlet, and reacting the liquid with the reagent in the assay chamber. Additionally, method 700 optionally includes applying a force to the assay chamber, and conveying, by the channel, the liquid from the assay chamber to the reservoir responsive to application of the force. The force optionally can include a centrifugal force. For example, the reservoir optionally can be disposed in a rotatable disc, wherein applying the force includes generating the centrifugal force by rotating the disc. As another example, the force optionally can be applied via a gas.

Optionally, in the device used in method 700, the assay chamber includes a lower assay chamber surface, an upper assay chamber surface, and an assay chamber sidewall extending between the upper and lower assay chamber surfaces. The assay chamber sidewall optionally includes a first portion extending substantially perpendicularly to the upper and lower assay chamber surfaces. The assay chamber sidewall includes a second portion extending at an obtuse angle, from the lower assay chamber surface. Optionally, method 700 includes, responsive to application of the force, the liquid being conveyed upward along the second portion and into the channel.

Additionally, or alternatively, in the device used in method 700 the assay chamber sidewall and the reservoir sidewall optionally are integrally formed with one another. As a further option, the upper assay chamber surface and the upper reservoir surface can be integrally formed with one another and attached to the integrally formed assay chamber sidewall and the reservoir sidewall. Optionally, the lower assay chamber surface and the lower reservoir surface are integrally formed with one another and attached to the integrally formed assay chamber sidewall and the reservoir sidewall. In various optional configurations of the device used in method 700, the assay chamber sidewall, the channel, and the reservoir sidewall can be integrally formed with one another. In various optional configurations of the device used in method 700, the assay chamber sidewall and the reservoir sidewall can be discrete elements. In various optional configurations of the device used in method 700, the upper assay chamber surface and the upper reservoir surface can be discrete elements. In various optional configurations of the device used in method 700, the lower assay chamber surface and the lower reservoir surface can be discrete elements. In various optional configurations of the device used in method 700, the assay chamber sidewall, the channel, and the reservoir sidewall can be discrete elements.

Optionally, method 700 includes generating the light of operation 720. Optionally, the light can be generated by a laser, light emitting diode, or lamp. Optionally, method 700 includes transmitting the light into the column through the upper reservoir surface. Method 700 further includes transmitting the light through the column and then through the lower reservoir surface. Alternatively, method 700 optionally can include transmitting the light into the column through the lower reservoir surface. As a further option, method 700 can include transmitting the light through the column and then through the upper reservoir surface.

Additionally, or alternatively, method 700 further can include receiving, by a sensor, the light transmitted through the at least one of the upper reservoir surface and the lower reservoir surface. For example, the sensor optionally receives the light through the upper reservoir surface. As another example, the sensor optionally receives the light through the lower reservoir surface. Additionally, or alternatively, optionally the light is generated by fluorescence or chemiluminescence

Note that devices such as described herein with reference to FIGS. 1A-6 and methods such as described herein with reference to FIG. 7 suitably can be used to read out information from any type of liquid. One nonlimiting example of a liquid is a bodily fluid, such as whole blood, blood plasma, blood cells, urine, or spit. Other nonlimiting examples of a liquid include a food sample or a water sample. In yet another example, the liquid can include a purified nucleic acid. In still another example, the liquid can include a pharmaceutical compound. Additionally, or alternatively, the liquid can include a buffer or reagent. Such buffer or reagent optionally can be mixed with one or more other liquids such as exemplified herein. In one specific, nonlimiting example, the liquid includes blood plasma which is mixed with a buffer and with a reagent within an assay chamber such as described herein with reference to FIGS. 3A-3B, 4A, or 5A-5C prior to using centrifugal force to move the mixture into a read well for optical analysis.

The present devices can be constructed using any suitable materials or combination of materials, such as any suitable combination of polymer, glass, metal, and semiconductor. Additionally, the present devices can be constructed using any suitable fabrication technique(s), such as molding, 3D printing, machining, laminate assemblies, thermoforming, chemical or laser etching, casting, and/or hot embossing.

It will be appreciated that the current subject matter provides many advantages. For example, the designs provided herein can limit the rate of evaporation by restricting the surface area of the fluid that is in contact with air. In particular, the current designs can constrict the air interface (meniscus) to the channel or to another area outside the reservoir such as the well.

As another example, the liquid reservoir designs provided herein can limit the movement of beads (used to capture analytes such as small molecules, proteins, nucleic acids, etc.) in solution when the reservoir is filled with fluid. Such an arrangement is advantageous for imaging purposes as it is desirable for the beads to not move during the imaging process. Beads in solution in the read chamber will settle over time to partially cover the bottom surface of the reservoir. Given that the bead solution is incompressible, and there is no head room in the reservoir, the fluid and beads in solution do not substantially move when the liquid reservoir is spun (i.e., by centrifugal force, etc.) or is otherwise agitated. This arrangement allows for beads in solution within the reservoir to be effectively imaged even when a device including such reservoir (e.g., disc-shaped cassette, etc.) is in motion.

In the descriptions above and in the claims, phrases such as "at least one of" or "one or more of" may occur followed by a conjunctive list of elements or features. The term "and/or" may also occur in a list of two or more elements or features. Unless otherwise implicitly or explicitly contradicted by the context in which it is used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." In addition, use of the term "based on," above and in the claims is intended to mean, "based at least in part on," such that an unrecited feature or element is also permissible.

The subject matter described herein can be embodied in systems, apparatus, methods, and/or articles depending on the desired configuration. The implementations set forth in the foregoing description do not represent all implementations consistent with the subject matter described herein. Instead, they are merely some examples consistent with aspects related to the described subject matter. Although a few variations have been described in detail above, other modifications or additions are possible. In particular, further features and/or variations can be provided in addition to those set forth herein. For example, the implementations described above can be directed to various combinations and subcombinations of the disclosed features and/or combinations and subcombinations of several further features disclosed above. In addition, the logic flows depicted in the accompanying figures and/or described herein do not necessarily require the particular order shown, or sequential order, to achieve desirable results. Other implementations may be within the scope of the following claims.

## Claims

1. A device (500) comprising:
a lower reservoir surface;
an upper reservoir surface, wherein the upper reservoir surface is without an opening; and
a reservoir sidewall (530) extending between the upper and lower reservoir surfaces,
wherein:
the lower reservoir surface, the upper reservoir surface, and the reservoir sidewall define a reservoir (540),
the reservoir is configured to be completely filled by a liquid such that the liquid forms a column contacting the upper reservoir surface, the lower reservoir surface, and the reservoir sidewall, with a meniscus of the liquid being outside of the reservoir, and
at least one of the upper reservoir surface and the lower reservoir surface is configured to transmit light, the device further comprising:
an assay chamber (541) fluidically coupled to the reservoir via a channel (560), wherein the channel is coupled to the reservoir sidewall (530);
wherein the assay chamber (541) includes a lower assay chamber surface (511), an upper assay chamber surface, and an assay chamber sidewall (531) extending between the upper and lower assay chamber surfaces;
wherein the channel (560) is located adjacent to the upper reservoir surface and the upper assay chamber surface;
wherein the assay chamber sidewall (531) includes a portion (533) extending at an obtuse angle from the lower assay chamber surface (511) and towards the channel (560); and
wherein the assay chamber (541) includes an inlet.

2. The device of any preceding claim, wherein the channel is configured to convey liquid from the assay chamber to the reservoir responsive to application of a force to the assay chamber.

3. The device of claim 2, wherein the force comprises a centrifugal force and the device further comprises a rotatable disc in which the reservoir is disposed, wherein rotating the disc generates the centrifugal force; or
wherein the device further comprises a source of gas and is configured to apply the force via the gas.

4. The device of any preceding claim, wherein the assay chamber sidewall includes a further portion extending substantially perpendicularly to the upper and lower assay chamber surfaces, and wherein the sidewall is optionally configured, responsive to application of the force, to convey the liquid upward along the obtuse sidewall portion and into the channel.

5. The device of any preceding claim, wherein the assay chamber sidewall and the reservoir sidewall are integrally formed with one another, and/or wherein the channel is integrally formed with the assay chamber sidewall and the reservoir sidewall.

6. The device of claim 5, wherein the upper assay chamber surface and the upper reservoir surface are integrally formed with one another and attached to the integrally formed assay chamber sidewall and the reservoir sidewall; and/or wherein the lower assay chamber surface and the lower reservoir surface are integrally formed with one another and attached to the integrally formed assay chamber sidewall and the reservoir sidewall.

7. The device of any of the preceding claims, further comprising a source of the light, wherein the source of the light comprises a laser, light emitting diode, or lamp, or is generated by fluorescence or chemiluminescence.

8. The device of claim 7, wherein the source of the light is positioned over the upper reservoir surface, is configured to transmit the light through the upper reservoir surface, and optionally is further configured to transmit the light through the column and then through the lower reservoir surface; or
wherein the source of the light is positioned under the lower reservoir surface, is configured to transmit the light through the lower reservoir surface, and optionally is further configured to transmit the light through the column and then through the upper reservoir surface.

9. The device of any of the preceding claims, further comprising a sensor configured to receive light transmitted through the at least one of the upper reservoir surface and the lower reservoir surface;
wherein the sensor is positioned over the upper reservoir surface and is configured to receive the light through the upper reservoir surface; or
wherein the sensor is positioned under the lower reservoir surface and is configured to receive the light through the lower reservoir surface.

10. The device of any of the preceding claims, wherein the reservoir has a volume of about 1-200 µL, or about 10-100 µL, or about 15-50 µL, or about 10-30 µL, or about 5-20 µL.

11. A method (700) of using a device according to any preceding claim, the method comprising:
completely filling the reservoir with a liquid such that a meniscus of the liquid is located outside of the reservoir (710); and
transmitting light through the at least one of the upper reservoir surface and the lower reservoir surface (720).

## Patentansprüche

1. Vorrichtung (500), die Folgendes umfasst:
eine untere Behälteroberfläche;
eine obere Behälteroberfläche, wobei die obere Behälteroberfläche ohne Öffnung ist; und
eine Behälterseitenwand (530), die sich zwischen der oberen und der unteren Behälteroberfläche erstreckt,
wobei:
die untere Behälteroberfläche, die obere Behälteroberfläche und die Behälterseitenwand einen Behälter (540) definieren,
der Behälter konfiguriert ist, mit einer Flüssigkeit vollständig gefüllt zu sein, derart, dass die Flüssigkeit eine Säule bildet, die die obere Behälteroberfläche, die untere Behälteroberfläche und die Behälterseitenwand berührt, wobei ein Meniskus der Flüssigkeit außerhalb des Behälters liegt, und
mindestens eine der oberen Behälteroberfläche und der unteren Behälteroberfläche konfiguriert ist, Licht weiterzuleiten, wobei die Vorrichtung ferner Folgendes umfasst:
eine Prüfkammer (541), die mittels eines Kanals (560) an den Behälter strömungstechnisch gekoppelt ist, wobei der Kanal an die Behälterseitenwand (530) gekoppelt ist;
wobei die Prüfkammer (541) eine untere Prüfkammeroberfläche (511), eine obere Prüfkammeroberfläche und eine Prüfkammerseitenwand (531), die sich zwischen der oberen und der unteren Prüfkammeroberfläche erstreckt, enthält;
der Kanal (560) benachbart zu der oberen Behälteroberfläche und der oberen Prüfkammeroberfläche angeordnet ist;
die Prüfkammerseitenwand (531) einen Abschnitt (533) enthält, der sich in einem stumpfen Winkel von der unteren Prüfkammeroberfläche (511) und zum Kanal (560) erstreckt; und
die Prüfkammer (541) einen Einlass enthält.

2. Vorrichtung nach einem vorhergehenden Anspruch, wobei der Kanal konfiguriert ist, in Reaktion auf das Ausüben einer Kraft auf die Prüfkammer eine Flüssigkeit von der Prüfkammer zum Behälter zu befördern.

3. Vorrichtung nach Anspruch 2, wobei die Kraft eine Zentrifugalkraft umfasst, die Vorrichtung ferner eine drehbare Scheibe umfasst, in der der Behälter angeordnet ist, und das Drehen der Scheibe die Zentrifugalkraft erzeugt; oder
die Vorrichtung ferner eine Gasquelle umfasst und konfiguriert ist, die Kraft mittels des Gases auszuüben.

4. Vorrichtung nach einem vorhergehenden Anspruch, wobei die Prüfkammerseitenwand einen weiteren Abschnitt enthält, der sich zu der oberen und der unteren Prüfkammeroberfläche im Wesentlichen senkrecht erstreckt, und die Seitenwand wahlweise konfiguriert ist, in Reaktion auf das Ausüben der Kraft die Flüssigkeit entlang des stumpfwinkligen Seitenwandabschnitts nach oben und in den Kanal zu befördern.

5. Vorrichtung nach einem vorhergehenden Anspruch, wobei die Prüfkammerseitenwand und die Behälterseitenwand miteinander einteilig gebildet sind und/oder der Kanal mit der Prüfkammerseitenwand und der Behälterseitenwand einteilig gebildet ist.

6. Vorrichtung nach Anspruch 5, wobei die obere Prüfkammeroberfläche und die obere Behälteroberfläche miteinander einteilig gebildet sind und an der einteilig gebildeten Prüfkammerseitenwand und der Behälterseitenwand angebracht sind und/oder die unter Prüfkammeroberfläche und die untere Behälteroberfläche miteinander einteilig gebildet sind und an der einteilig gebildeten Prüfkammerseitenwand und der Behälterseitenwand angebracht sind.

7. Vorrichtung nach einem der vorhergehenden Ansprüche, die ferner eine Lichtquelle umfasst, wobei die Lichtquelle einen Laser, eine Leuchtdiode oder eine Lampe umfasst oder durch Fluoreszenz oder Chemolumineszenz erzeugt wird.

8. Vorrichtung nach Anspruch 7, wobei die Lichtquelle über der oberen Behälteroberfläche positioniert ist, konfiguriert ist, das Licht durch die obere Behälteroberfläche weiterzuleiten, und wahlweise ferner konfiguriert ist, das Licht durch die Säule und dann durch die untere Behälteroberfläche weiterzuleiten; oder die Lichtquelle unter der unteren Behälteroberfläche positioniert ist, konfiguriert ist, das Licht durch die untere Behälteroberfläche weiterzuleiten, und wahlweise ferner konfiguriert ist, das Licht durch die Säule und dann durch die obere Behälteroberfläche weiterzuleiten.

9. Vorrichtung nach einem der vorhergehenden Ansprüche, die ferner einen Sensor umfasst, der konfiguriert ist, Licht zu empfangen, das durch die mindestens eine der oberen Behälteroberfläche und der unteren Behälteroberfläche weitergeleitet wird;
wobei der Sensor über der oberen Behälteroberfläche positioniert ist und konfiguriert ist, das Licht durch die obere Behälteroberfläche zu empfangen; oder
der Sensor unter der unteren Behälteroberfläche positioniert ist und konfiguriert ist, das Licht durch die untere Behälteroberfläche zu empfangen.

10. Vorrichtung nach einem der vorhergehenden Ansprüche, wobei der Behälter ein Volumen von etwa 1-200 µL oder etwa 10-100 µL oder etwa 15-50 µL oder etwa 10-30 µL oder etwa 5-20 µL aufweist.

11. Verfahren (700), das eine Vorrichtung nach einem vorhergehenden Anspruch verwendet, wobei das Verfahren Folgendes umfasst:
vollständiges Füllen des Behälters mit einer Flüssigkeit, derart, dass ein Meniskus der Flüssigkeit sich außerhalb des Behälters (710) befindet; und
Weiterleiten von Licht durch die mindestens eine der oberen Behälteroberfläche und der unteren Behälteroberfläche (720).

## Revendications

1. Dispositif (500), comprenant :
une surface de réservoir inférieure ;
une surface de réservoir supérieure, dans lequel la surface de réservoir supérieure est sans ouverture ; et
une paroi latérale de réservoir (530) s'étendant entre les surfaces de réservoir supérieure et inférieure, dans lequel :
la surface de réservoir inférieure, la surface de réservoir supérieure, et la paroi latérale de réservoir définissent un réservoir (540),
le réservoir est configuré pour être complètement rempli par un liquide de manière telle que le liquide forme une colonne entrant en contact avec la surface de réservoir supérieure, la surface de réservoir inférieure, et la paroi latérale de réservoir, un ménisque du liquide étant à l'extérieur du réservoir, et
au moins une de la surface de réservoir supérieure et de la surface de réservoir inférieure est configurée pour transmettre de la lumière, le dispositif comprenant en outre :
une chambre d'analyse (541) couplée fluidiquement au réservoir par l'intermédiaire d'un canal (560), dans lequel le canal est couplé à la paroi latérale de réservoir (530) ;
dans lequel la chambre d'analyse (541) inclut une surface de chambre d'analyse inférieure (511), une surface de chambre d'analyse supérieure, et une paroi latérale de chambre d'analyse (531) s'étendant entre les surfaces de chambre d'analyse supérieure et inférieure ;
dans lequel le canal (560) est situé de façon adjacente à la surface de réservoir supérieure et à la surface de chambre d'analyse supérieure ;
dans lequel la paroi latérale de chambre d'analyse (531) inclut une partie (533) s'étendant à un angle obtus depuis la surface de chambre d'analyse inférieure (511) et vers le canal (560) ; et
dans lequel la chambre d'analyse (541) inclut une entrée.

2. Dispositif d'une quelconque revendication précédente, dans lequel le canal est configuré pour transporter un liquide depuis la chambre d'analyse jusqu'au réservoir en réponse à une application d'une force sur la chambre d'analyse.

3. Dispositif de la revendication 2, dans lequel la force comprend une force centrifuge et le dispositif comprend en outre un disque rotatif dans lequel le réservoir est disposé, dans lequel la mise en rotation du disque génère la force centrifuge ; ou
dans lequel le dispositif comprend en outre une source de gaz et est configuré pour appliquer la force par l'intermédiaire du gaz.

4. Dispositif d'une quelconque revendication précédente, dans lequel la paroi latérale de chambre d'analyse inclut une partie supplémentaire s'étendant de façon sensiblement perpendiculaire aux surfaces de chambre d'analyse supérieure et inférieure, et dans lequel la paroi latérale est facultativement configurée, en réponse à une application de la force, pour transporter le liquide vers le haut le long de la partie de paroi latérale obtuse et jusque dans le canal.

5. Dispositif d'une quelconque revendication précédente, dans lequel la paroi latérale de chambre d'analyse et la paroi latérale de réservoir sont formées de façon monobloc l'une avec l'autre, et/ou dans lequel le canal est formé de façon monobloc avec la paroi latérale de chambre d'analyse et la paroi latérale de réservoir.

6. Dispositif de la revendication 5, dans lequel la surface de chambre d'analyse supérieure et la surface de réservoir supérieure sont formées de façon monobloc l'une avec l'autre et fixées à la paroi latérale de chambre d'analyse et à la paroi latérale de réservoir formées de façon monobloc ; et/ou dans lequel la surface de chambre d'analyse inférieure et la surface de réservoir inférieure sont formées de façon monobloc l'une avec l'autre et fixées à la paroi latérale de chambre d'analyse et à la paroi latérale de réservoir formées de façon monobloc.

7. Dispositif de quelconques des revendications précédentes, comprenant en outre une source de la lumière, dans lequel la source de la lumière comprend un laser, une diode électroluminescente, ou une lampe, ou est générée par fluorescence ou chimioluminescence.

8. Dispositif de la revendication 7, dans lequel la source de la lumière est positionnée au-dessus de la surface de réservoir supérieure, est configurée pour transmettre la lumière à travers la surface de réservoir supérieure, et facultativement est en outre configurée pour transmettre la lumière à travers la colonne et puis à travers la surface de réservoir inférieure ; ou
dans lequel la source de la lumière est positionnée en dessous de la surface de réservoir inférieure, est configurée pour transmettre la lumière à travers la surface de réservoir inférieure, et facultativement est en outre configurée pour transmettre la lumière à travers la colonne et puis à travers la surface de réservoir supérieure.

9. Dispositif de quelconques des revendications précédentes, comprenant en outre un capteur configuré pour recevoir de la lumière transmise à travers l'au moins une de la surface de réservoir supérieure et de la surface de réservoir inférieure ;
dans lequel le capteur est positionné au-dessus de la surface de réservoir supérieure et est configuré pour recevoir la lumière à travers la surface de réservoir supérieure ; ou
dans lequel le capteur est positionné en dessous de la surface de réservoir inférieure et est configuré pour recevoir la lumière à travers la surface de réservoir inférieure.

10. Dispositif de quelconques des revendications précédentes, dans lequel le réservoir a un volume d'environ 1 à 200 µL, ou d'environ 10 à 100 µL, ou d'environ 15 à 50 µL, ou d'environ 10 à 30 µL, ou d'environ 5 à 20 uL.

11. Procédé (700) d'utilisation d'un dispositif selon une quelconque revendication précédente, le procédé comprenant :
le remplissage complet du réservoir avec un liquide de manière telle qu'un ménisque du liquide est situé à l'extérieur du réservoir (710) ; et
la transmission de lumière à travers l'au moins une de la surface de réservoir supérieure et de la surface de réservoir inférieure (720).
